# DEMANDE DE BREVET EUROPEEN

(11) **EP 2 156 818 A2**
(43) Date de publication de la demande: **24.02.2010**
(21) Numéro de dépôt: 09166023.3
(22) Date de dépôt: 21.07.2009
(51) Int. Cl.: A61K 8/31, A61K 8/19, A61K 8/92, A61K 8/06, A61Q 1/02

(54) **Compositions cosmetique comprenant du carbonate de calcium**

(30) Priorité: 21.07.2008 FR 0854940
(71) Demandeur: L'Oréal, 75008 Paris (FR)
(72) Inventeur: Clavel, Euriel, 75013, Paris (FR); Arnaud, Pascal, 94240, L'Haye-les-Roses (FR)
(74) Mandataire: Le Coupanec, Pascale A.M.P.

(57) **Abrégé**

La présente invention concerne des compositions cosmétiques sous forme d'une émulsion comprenant au moins une phase aqueuse et au moins une phase grasse, la phase grasse contient au moins une huile végétale, et la composition comprend au moins un solvant volatil de type alcane(s) linéaire(s) volatil(s) en C₉ à C₁₅ et au moins un carbonate de calcium.

## Description

La présente invention a trait à des compositions cosmétiques, notamment sous forme d'émulsions, pour le maquillage et/ou le soin des matières kératiniques, notamment la peau et les lèvres.

En particulier, la présente invention concerne des émulsions comprenant au moins une huile végétale.

La mise en oeuvre d'huiles végétales dans la formulation de compositions cosmétiques est connue depuis l'antiquité. Ces huiles sont dotées de propriétés émollientes, hydratantes et permettent de conférer à la peau une grande douceur.

Toutefois, les huiles végétales, du fait de leur polarité élevée, peuvent diminuer la stabilité des émulsions.

Qui plus est, ces huiles peuvent donner aux compositions qui les renferment un caractère gras et collant excessif, pendant et après leur application, notamment lorsqu'elles sont présentes en une teneur élevée. En outre, elles peuvent être à l'origine d'un effet brillant, qui peut s'avérer néfaste aux propriétés cosmétiques recherchées, notamment dans le cadre du soin ou du maquillage de la peau, comme avec les fonds de teint.

Les inconvénients mentionnés ci-dessus peuvent être d'autant plus prononcés que la composition cosmétique comprend une phase grasse externe, comme par exemple les émulsions eau-dans-huile E/H.

Pour pallier ces inconvénients, il a déjà été proposé d'associer à ces huiles végétales, des composés volatils, comme les silicones cycliques ou l'isododécane, de manière à diminuer leur caractère gras et collant et de favoriser ainsi l'application des compositions cosmétiques.

Cependant, cette solution ne se révèle pas toujours satisfaisante, notamment lors de l'utilisation de matières premières naturelles ou d'origine naturelle pour formuler les compositions cosmétiques.

Or, parallèlement, les consommatrices sont de plus en plus à la recherche de produits cosmétiques formés, en tout ou partie, de constituants végétaux ou d'origine végétale.

Par exemple, la demande WO 2007/068371 décrit des compositions cosmétiques comprenant des alcanes d'origine végétale.

Ainsi, il existe un besoin de pouvoir formuler des compositions cosmétiques, notamment sous forme d'émulsions, comprenant une huile végétale et comprenant une teneur réduite, voire étant dépourvue de silicones cycliques ou de dérivés pétrochimiques, tel que l'isododécane.

Il existe également un besoin de pouvoir proposer des compositions cosmétiques, notamment sous forme d'émulsions, comprenant une huile végétale, et présentant des propriétés satisfaisantes, voire améliorées, tant sur le plan cosmétique que sur le plan de la stabilité.

Il existe également un besoin de proposer des compositions cosmétiques, notamment sous forme d'émulsions, confortables à l'application, et, notamment, présentant peu ou pas de caractère gras et/ou collant.

Il existe encore un besoin de disposer de compositions cosmétiques, notamment sous forme d'émulsions, en particulier pour le soin ou le maquillage de la peau, comprenant une huile végétale et présentant peu ou pas d'effet brillant.

Enfin, il existe encore un besoin de disposer de compositions cosmétiques comprenant une phase grasse externe, par exemple des émulsions E/H présentant des propriétés cosmétiques, notamment émollientes et/ou hydratantes, et une stabilité améliorées, ainsi qu'un faible, voire pas de caractère gras et/ou collant.

La présente invention a pour objet de satisfaire à ces besoins.

Ainsi, selon un de ces premiers objets, l'invention concerne une composition cosmétique sous forme d'une émulsion comprenant au moins une phase aqueuse et au moins une phase grasse, ladite phase grasse contenant au moins une huile végétale, et ladite composition comprenant en outre au moins un alcane linéaire volatil et/ou un solvant volatil de type alcane(s) linéaire(s) volatil(s) en C₉ à C₁₅ et au moins un carbonate de calcium.

Selon un autre de ses objets, l'invention concerne une composition cosmétique sous forme d'une émulsion comprenant au moins une phase aqueuse et au moins une phase grasse, ladite émulsion étant une émulsion eau-dans-huile E/H, ladite phase grasse contenant au moins une huile végétale, et ladite composition comprenant en outre au moins un alcane linéaire volatil et/ou un solvant volatil de type alcane(s) linéaire(s) volatil(s) en C₉ à C₁₅ et au moins un carbonate de calcium.

De manière inattendue, les inventeurs ont observé que l'association d'au moins un alcane linéaire volatil spécifique et/ou un solvant volatil de type alcane(s) linéaire(s) volatil(s) en C₉ à C₁₅, par exemple le n-undécane, le n-tridécane, ou un de leurs mélanges, avec au moins une charge telle que du carbonate de calcium, permet de formuler des émulsions, en particulier des émulsions E/H, comprenant au moins une huile végétale et présentant des propriétés satisfaisantes tant sur le plan cosmétique que sur le plan de la stabilité.

L'association d'au moins un alcane linéaire volatil spécifique et/ou un solvant volatil de type alcane(s) linéaire(s) volatil(s) en C₉ à C₁₅, ou un de leurs mélanges, avec au moins une charge telle que du carbonate de sodium, permet avantageusement de stabiliser des émulsions de type eau-dans-huile E/H.

Selon une variante de réalisation, l'alcane linéaire volatil et/ou le solvant volatil de type alcane(s) linéaire(s) volatil(s) en C₉ à C₁₅ est/sont d'origine végétale.

Au sens de l'invention, on entend par « alcane linéaire » un alcane linéaire non ramifié par opposition aux alcanes ramifiés et aux cycloalcanes.

Au sens de l'invention, on entend par « composé d'origine végétale », un composé issu d'un végétal et ayant subi une ou plusieurs modifications chimiques par exemple par réaction de synthèse organique.

Au sens de l'invention, on entend par « composé végétal », un composé immédiatement issu d'un végétal sans avoir subi de modification chimique.

Ainsi, comme illustré par les exemples, les inventeurs ont observé qu'il est possible d'améliorer la stabilité d'une émulsion, en particulier d'une émulsion E/H, comprenant à titre d'huile végétale de l'huile de macadamia ou de l'huile de jojoba, éventuellement en mélange avec de l'huile de soja, en y introduisant un mélange de n-undécane et de n-tridécane et du carbonate de calcium.

En outre, les compositions de l'invention présentent avantageusement peu, voire pas d'effet collant et/ou gras à l'application.

Enfin, les compositions de l'invention présentent un effet brillant significativement réduit et donc n'affectant pas les propriétés cosmétiques, et notamment esthétiques, des compositions de soin ou de maquillage. Notamment, les compositions cosmétiques de l'invention ne présentent pas d'effet brillant.

Selon un autre de ses aspects, la présente invention concerne l'utilisation d'au moins un alcane linéaire volatil et/ou d'un solvant volatil de type alcane(s) linéaire(s) volatil(s) en C₉ à C₁₅, en association avec un carbonate de calcium dans une émulsion comprenant au moins une huile végétale, pour conférer une stabilité améliorée à ladite émulsion.

Selon un autre de ses aspects, la présente invention concerne l'utilisation d'au moins un alcane linéaire volatil et/ou d'un solvant volatil de type alcane(s) linéaire(s) volatil(s) en C₉ à C₁₅, en association avec un carbonate de calcium dans une émulsion E/H comprenant au moins une huile végétale, pour conférer une stabilité améliorée à ladite émulsion E/H.

La présente invention a également pour objet un procédé cosmétique de maquillage et/ou de soin de matières kératiniques comprenant au moins l'application sur lesdites matières d'au moins une couche d'une composition selon l'invention.

Selon une variante de réalisation, un procédé cosmétique de maquillage de l'invention peut également être effectué sur un support synthétique.

Une composition de l'invention est une émulsion possédant une phase continue huileuse ou aqueuse. Une telle composition peut être, par exemple, une émulsion inverse (E/H) ou directe (H/E), ou encore une émulsion multiple (E/H/E ou H/E/H).

Selon une variante de réalisation, une émulsion selon l'invention est avantageusement une émulsion de type E/H.

La présente invention concerne des compositions cosmétiques pouvant se présenter sous la forme d'une composition dermatologique ou d'une composition de soin des matières kératiniques, notamment de la peau ou des lèvres, ou encore sous la forme d'une composition de protection solaire. Elle peut se présenter alors sous une forme non colorée, contenant éventuellement des actifs cosmétiques ou dermatologiques. Elle peut alors être utilisée comme base de soin pour les matières kératiniques, notamment la peau ou les lèvres.

Une composition de l'invention peut également se présenter sous forme d'un produit coloré de maquillage des matières kératiniques, notamment pour le maquillage de la peau ou des lèvres, tel qu'un fond de teint, un blush, un fard à joues ou à paupières, un produit de coloration de la peau, un produit anticerne, un rouge à lèvres, un baume à lèvres, ou encore de maquillage des cils ou des sourcils comme un mascara.

Selon l'invention, on entend désigner par matières kératiniques, la peau du corps et les muqueuses, par exemple le visage ou les lèvres, ainsi que les poils et les cheveux, et notamment les cils.

Outre les composés précédemment mentionnés, les composés ou additifs additionnels entrant dans la formulation de l'invention sont de préférence naturels ou d'origine naturelle.

Par « composé naturel », on entend un composé que l'on obtient directement de la terre ou du sol, ou à partir de végétaux ou d'animaux, *via*, le cas échéant, un ou des processus physiques, comme par exemple un broyage, un raffinage, une distillation, une purification ou une filtration.

Par composés « d'origine naturelle », on entend un composé naturel ayant subi un ou des traitements chimiques ou industriels annexes, engendrant des modifications n'affectant pas les qualités essentielles de ce composé et/ou un composé comprenant majoritairement des constituants naturels ayant ou non subi des transformations, comme indiquées ci-dessus.

A titre d'exemple non limitatif de traitement chimique ou industriel annexe engendrant des modifications n'affectant pas les qualités essentielles d'un composé naturel, on peut mentionner ceux autorisés par les organismes de contrôle tels qu'Ecocert (Référentiel des produits cosmétiques biologiques et écologiques, janvier 2003) ou définis dans les manuels reconnus dans le domaine, tels que « Cosmetics and Toiletries Magazine », 2005, vol. 120, 9:10.

Au sens de l'invention, on entend par « composé synthétique ou artificiel », un composé ne satisfaisant pas aux définitions des composés naturels ou d'origine naturelle indiquées ci-dessus.

### STABILITE

La stabilité d'une composition de l'invention, et notamment d'une émulsion, peut être évaluée au moyen du protocole suivant.

Une composition est préparée puis disposée dans une étuve à cycles, par exemple une étuve de marque Vôtch VT4004.

La composition est soumise à un ensemble de cycles successifs de -20 °C à +20 °C. Un ensemble d'au moins 10 cycles est effectué.

Chacun des cycles dure 24 heures et comprend les étapes suivantes 6 heures à 20 °C, puis 6 heures de descente en température jusqu'à -20 °C, puis 6 heures à une température de - 20 °C, et enfin 6 heures de remontée en température jusqu'à 20 °C.

Après chaque cycle, les aspects macroscopiques et microscopiques de la composition sont évalués.

Au bout de 10 cycles, la composition ne doit pas présenter de modifications d'aspect macroscopique : elle doit rester lisse et homogène, sans relargage, sans séparation de phase et sans changement de couleur.

La composition est observée au microscope entre lame et lamelle, à un grossissement X10. Son aspect microscopique doit rester proche de l'aspect initial.

On ne doit pas observer, en particulier, de dégradation de l'émulsion (fond d'émulsion plus grossier, coalescence traduite par la présence de nombreuses grosses gouttes, modification des bords de préparation, présence de cristaux).

### SOLVANT VOLATIL DE TYPE ALCANE(S) LINEAIRE(S) VOLATIL(S)

Une composition selon l'invention comprend au moins un alcane linéaire volatil et/ou un solvant volatil de type alcane(s) linéaire(s) volatil(s), les alcanes linéaires volatils étant comme défini ci-après.

Au sens de l'invention, on entend par solvant volatil de type alcane(s) linéaire(s) volatil(s), un solvant comprenant un unique composé de type alcane linéaire volatil ou un mélange de composés de type alcane(s) linéaire(s) volatil(s).

Un solvant volatil de type alcane(s) linéaire(s) volatil(s) est pour l'essentiel composé d'alcane(s) linéaire(s) volatil(s) comme décrits ci-après.

Par « pour l'essentiel », on entend que le solvant volatil de type alcane(s) linéaire(s) volatil(s) comprend au moins 80 % en poids, de préférence au moins 90 % en poids et mieux au moins 95 % voire au moins 98 % en poids d'alcane(s) linéaire(s) volatil(s) par rapport au poids total d'hydrocarbures dans ledit solvant.

Un alcane linéaire volatil et/ou un solvant volatil de type alcane(s) linéaire(s) volatil(s) entre(ent) dans la composition d'une phase grasse liquide d'une composition selon l'invention, notamment dans la phase d'huile(s) hydrocarbonée(s), et plus particulièrement dans la phase d'huile(s) d'hydrocarbures.

Selon un mode de réalisation, un alcane linéaire volatil et/ou un solvant volatil de type alcane(s) linéaire(s) volatil(s) convenant à l'invention peut présenter un point éclair compris dans l'intervalle variant de 70 à 120 °C, et plus particulièrement de 80 à 100 °C, et notamment d'être d'environ 89 °C.

Un alcane linéaire volatil convenant à l'invention est liquide à température ambiante (environ 25 °C).

Selon un mode de réalisation, un solvant volatil de type alcane(s) linéaire(s) volatil(s) convenant à l'invention peut comprendre au moins un alcane linéaire volatil comprenant de 9 à 15 atomes de carbone, en particulier de 10 à 15 atomes de carbone, et plus particulièrement de 11 à 13 atomes de carbone.

Un alcane linéaire volatil et/ou un solvant volatil de type alcane(s) linéaire(s) volatil(s) en C₉ à C₁₅ convenant à l'invention peut/peuvent avantageusement être d'origine végétale.

Un tel alcane peut être obtenu, directement ou en plusieurs étapes, à partir d'une matière première végétale comme une huile, un beurre, une cire, etc.

A titre d'exemple d'alcane convenant à l'invention, on peut mentionner les alcanes décrits dans la demande de brevet de la société Cognis WO 2007/068371.

Ces alcanes sont obtenus à partir d'alcools gras, eux-mêmes obtenus à partir d'huile de coprah ou de palme.

A titre d'exemple de solvant volatil de type alcane(s) linéaire(s) volatil(s) convenant à l'invention, on peut citer les solvants comprenant au moins un alcane linéaire volatil choisi parmi le n-nonane (C9), le n-décane (C10), le n-undécane (C11), le n-dodécane (C12), le n-tridécane (C13), le n-tétradecane (C14) et le n-pentadécane (C15) , et leurs mélanges, et en particulier le mélange de n-undécane (C11) et de n-tridécane (C13) commercialisé sous la référence de CETIOL UT par la Société Cognis.

Selon un mode de réalisation particulier, un solvant volatil de type alcane(s) linéaire(s) volatil(s) convenant à l'invention on peut citer les solvants comprenant au moins un alcane linéaire volatil choisi parmi le n-nonane, le n-undécane, le n-dodécane, le n-tridécane, et leurs mélanges.

Plus particulièrement, un solvant volatil de type alcane(s) linéaire(s) volatil(s) convenant à l'invention peut être mis en oeuvre sous la forme d'un mélange n-undécane/n-tridécane.

De préférence, dans un tel mélange, le rapport pondéral n-undécane : n-tridécane peut être 50 : 50 à 90 : 10, de préférence variant de 60 : 40 à 80 : 20, de préférence variant de 65 : 35 à 75 : 25.

En particulier, une composition selon l'invention peut comprendre un mélange n-undécane : n-tridécane dans un rapport pondéral de 70 : 30.

Selon un mode de réalisation particulier, un alcane linéaire volatil et/ou un solvant volatil de type alcane(s) linéaire(s) volatil(s) convenant à l'invention peut présenter, à 25 °C, une vitesse d'évaporation inférieure ou égale à 0,13 mg/cm²/min.

Selon un mode de réalisation, un alcane linéaire volatil et/ou un solvant volatil de type alcane(s) linéaire(s) volatil(s) convenant à l'invention peut présenter, à 25 °C, une vitesse d'évaporation comprise dans l'intervalle variant de 0,05 à 0,13 mg/cm²/min, en particulier de 0,08 à 0,12 mg/cm²/min, et plus particulièrement de 0,1 à 0,12 mg/cm²/min.

La volatilité d'un alcane linéaire volatil et/ou d'un solvant volatil de type alcane(s) linéaire(s) volatil(s) conforme à l'invention peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m³ régulée en température (25 °C) et en hygrométrie (humidité relative 50 %) 15 g d'alcane linéaire volatil et/ou de solvant volatil de type alcane(s) linéaire(s) volatil(s).

On laisse le liquide s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant l'alcane linéaire volatil et/ou le solvant volatil de type alcane(s) linéaire(s) volatil(s), les pales étant dirigées vers le cristallisoir, à une distance de 20 cm par rapport au fond du cristallisoir.

On mesure à intervalles de temps réguliers la masse d'alcane linéaire volatil et/ou de solvant volatil de type alcane(s) linéaire(s) volatil(s) restante dans le cristallisoir.

Les vitesses d'évaporation sont exprimées en mg d'alcane linéaire volatil et/ou de solvant volatil de type alcane(s) linéaire(s) volatil(s) évaporé par unité de surface (cm²) et par unité de temps (minute).

L'alcane linéaire volatil et/ou le solvant volatil de type alcane(s) linéaire(s) volatil(s) selon l'invention a une vitesse d'évaporation inférieure ou égale à 0,13 mg/cm²/min. Cela correspond donc à une quantité de solvant évaporé inférieure ou égale à 3,9 mg/ cm² en 30 minutes.

Une composition de l'invention peut comprendre de 0,5 % à 90 % en poids d'alcane linéaire volatil et/ou de solvant volatil de type alcane(s) linéaire(s) volatil(s), en particulier de 1 à 50 % en poids, et plus particulièrement de 5 à 40 % en poids d'alcane linéaire volatil et/ou de solvant volatil de type alcane(s) linéaire(s) volatil(s) par rapport au poids total de la composition.

### CARBONATE DE CALCIUM

Une composition de l'invention comprend au moins un carbonate de calcium. Un carbonate de calcium convenant à l'invention peut être d'origine naturelle ou synthétique.

A titre d'exemples de source de carbonate de calcium naturel à mettre en oeuvre dans une composition de l'invention, on peut citer les craies, les calcaires, les marbres, et leurs mélanges.

On peut citer, par exemple, le carbonate de calcium naturel commercialisé sous la référence commerciale OMYAPURE 35 LM-OG par la société OMYA.

Les carbonates de calcium synthétiques peuvent être obtenus par un procédé de précipitation.

A titre d'exemples de carbonate de calcium synthétique convenant à l'invention, on peut citer, par exemple, le carbonate de calcium commercialisé sous la référence commerciale SOCAL 90 A ou SOCAL 2P par la société Solvay.

Un carbonate de calcium convenant à l'invention peut se présenter sous forme de particules présentant une taille moyenne pouvant varier de 0,1 à 20 µm, en particulier de 0,5 à 10 µm, et plus particulièrement de 1 à 5 µm.

Une composition de l'invention peut comprendre de 0,1 à 20 % en poids de carbonate de calcium, en particulier de 0,5 à 15 % de carbonate de calcium et plus particulièrement de 1 à 10 % en poids de carbonate de calcium par rapport au poids total de la composition.

### HUILES VEGETALES

Une composition de l'invention comprend au moins une huile végétale.

Une huile végétale peut être extraite d'un produit végétal soit par une seule pression à froid (huile vierge) soit par pression à chaud et raffinage (huile raffinée).

Les huiles végétales de l'invention sont des huiles extraites directement des végétaux sans subir de modification chimique.

A titre d'huile végétale convenant à l'invention, on peut citer l'huile d'aloès, l'huile d'amande douce, l'huile d'amande de pêche, l'huile d'arachide, l'huile d'argan, l'huile d'avocat, l'huile de bancoulier, l'huile de baobab, l'huile de bourrache, l'huile de brocoli, l'huile de calendula, l'huile de caméline, l'huile de carotte, l'huile de carthame, l'huile de chanvre, l'huile de colza, l'huile de coton, l'huile de coprah, l'huile de graine de courge, l'huile de germe de blé, l'huile de jojoba, l'huile de karité, l'huile de luzerne, l'huile de lys, l'huile de macadamia, l'huile de maïs, l'huile de meadowfoam, l'huile de millepertuis, l'huile de millet, l'huile de monoï, l'huile de noisette, l'huile de noyaux d'abricot, l'huile de noix, l'huile d'olive, l'huile d'onagre, l'huile d'orge, l'huile de palme, l'huile de passiflore, l'huile de pavot, l'huile de pépins de cassis, l'huile de pépins de kiwi, l'huile de pépins de raisin, l'huile de pistache, l'huile de potiron, l'huile de potimarron, l'huile de quinoa, l'huile de rosier muscat, l'huile de sésame, l'huile de seigle, l'huile de soja, l'huile de tournesol, l'huile de ricin, et l'huile de watermelon, et leurs mélanges.

Selon un mode de réalisation, une huile végétale convenant à l'invention peut en particulier être choisie parmi l'huile de macadamia, l'huile de jojoba, et leurs mélanges.

Une composition selon l'invention peut comprendre de 0,5 % à 80 % en poids d'huile végétale, par rapport au poids total de la composition, en particulier allant de 1 % à 50 % en poids d'huile végétale, et plus particulièrement allant de 2 % à 25 % en poids d'huile végétale, par rapport au poids total de la composition.

### AGENTS TENSIOACTIFS

Une composition selon l'invention comprend au moins un agent tensioactif, notamment choisi parmi les agents tensioactifs amphotères, anioniques, cationiques ou non ioniques, utilisés seuls ou en mélange.

Les agents tensioactifs sont généralement présents dans la composition, en une proportion pouvant aller par exemple de 0,3 à 20 % en poids, en particulier de 0,5 à 15 % en poids, et plus particulièrement de 1 à 10 % en poids d'agents tensioactifs par rapport au poids total de la composition.

Bien entendu, l'agent tensioactif est choisi de manière à stabiliser efficacement les émulsions plus particulièrement considérées selon l'invention à savoir de type H/E, E/H, ou H/E/H, et en particulier de type E/H. Ce choix relève des compétences de l'homme de l'art.

Pour les émulsions H/E, on peut citer par exemple les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés ; les éthers d'alcools gras; les éthers de sucre et d'alcools gras en C₈-C₂₄, et leurs mélanges.

Comme ester de glycéryle et d'acide gras, on peut citer notamment le stéarate de glycéryle (mono-, di- et/ou tri-stéarate de glycéryle) (nom CTFA : glycéryl stéarate) ou le ricinoléate de glycéryle, et leurs mélanges.

Comme ester de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom CTFA : PEG-50 stéarate), le monostéarate de polyéthylène glycol 100 OE (nom CTFA : PEG-100 stéarate) et leurs mélanges.

On peut aussi utiliser des mélanges de ces agents tensioactifs, comme par exemple le produit contenant du Glycéryl stéarate et du PEG-100 stéarate, commercialisé sous la dénomination ARLACEL 165 par la société Uniqema, et le produit contenant du Glycéryl stéarate (mono-distéarate de glycéryle) et du stéarate de potassium, commercialisé sous la dénomination TEGIN par la société Goldschmidt (nom CTFA : glyceryl stearate SE).

Comme ester d'acide gras et de glucose ou d'alkylglucose, on peut citer en particulier le palmitate de glucose, les sesquistéarates d'alkylglucose comme le sesquistéarate de méthylglucose, les palmitates d'alkylglucose comme le palmitate de méthylglucose ou d'éthylglucose, les esters gras de méthylglucoside et plus spécialement le diester de méthylglucoside et d'acide oléique (nom CTFA : Methyl glucose dioleate) ; l'ester mixte de méthylglucoside et du mélange acide oléique / acide hydroxystéarique (nom CTFA : Méthyl glucose dioleate/hydroxystearate) ; l'ester de méthylglucoside et d'acide isostéarique (nom CTFA : Méthyl glucose isostearate) ; l'ester de méthylglucoside et d'acide laurique (nom CTFA : Méthyl glucose laurate) ; le mélange de monoester et de diester de méthylglucoside et d'acide isostéarique (nom CTFA : Methyl glucose sesqui-isostéarate) ; le mélange de monoester et de diester de méthylglucoside et d'acide stéarique (nom CTFA : Méthyl glucose sesquistearate) et en particulier le produit commercialisé sous la dénomination Glucate SS par la société AMERCHOL, et leurs mélanges.

Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom CTFA : PEG-20 methyl glucose distéarate) tel que le produit commercialisé sous la dénomination Glucam E-20 distéarate par la société AMERCHOL ; l'éther de polyéthylène glycol du mélange de monoester et de diester de méthyl glucose et d'acide stéarique à environ 20 moles d'oxyde d'éthylène (nom CTFA : PEG-20 méthyl glucose sesquistéarate) et en particulier le produit commercialisé sous la dénomination Glucamate SSE-20 par la société AMERCHOL et celui commercialisé sous la dénomination Grillocose PSE-20 par la société GOLDSCHMIDT, et leurs mélanges.

Comme esters de sucrose, on peut citer par exemple le palmito-stéarate de saccharose, le stéarate de saccharose et le mono laurate de saccharose.

Comme éthers d'alcools gras, on peut citer par exemple les éthers de polyéthylène glycol et d'alcool gras comportant de 8 à 30 atomes de carbone, et notamment de 10 à 22 atomes de carbone, tels que les éthers de polyéthylène glycol et d'alcools cétylique, stéarylique, cetéarylique (mélange d'alcools cétylique et stéarylique). On peut citer par exemple les éthers comportant de 1 à 200 et de préférence de 2 à 100 groupes oxyéthylénés, tels que ceux de nom CTFA Ceteareth-20, Ceteareth-30, et leurs mélanges.

Comme éthers de sucre, on peut citer notamment les alkylpolyglucosides, et par exemple le décylglucoside comme le produit commercialisé sous la dénomination MYDOL 10 par la société Kao Chemicals, le produit commercialisé sous la dénomination PLANTAREN 2000 par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Henkel ; le coco-glucoside comme le produit commercialisé sous la dénomination PLANTACARE 818/UP par la société Henkel ; le cétostéaryl glucoside éventuellement en mélange avec l'alcool cétostéarylique, commercialisé par exemple sous la dénomination MONTANOV 68 par la société Seppic, sous la dénomination TEGO-CARE CG90 par la société Goldschmidt et sous la dénomination EMULGADE KE3302 par la société Henkel ; l'arachidyl glucoside, par exemple sous la forme du mélange d'alcools arachidique et béhénique et d'arachidyl glucoside commercialisé sous la dénomination MONTANOV 202 par la société Seppic ; le cocoyléthylglucoside, par exemple sous la forme du mélange (35/65) avec les alcools cétylique et stéarylique, commercialisé sous la dénomination MONTANOV 82 par la société Seppic, et leurs mélanges.

Pour les émulsions E/H, on peut utiliser des agents tensioactifs hydrocarbonés ou siliconés.

Selon une variante de réalisation, les tensioactifs hydrocarbonés sont préférés.

On peut citer par exemple comme agents tensioactifs hydrocarbonés, les polyesters de polyols comme le PEG-30 dipolyhydroxystearate vendu sous la référence ARLACEL P 135 par la société Uniqema, le polyglyceryl-2 dipolyhydroxystearate vendu sous la référence DEHYMULS PGPH par la société Cognis.

On peut citer par exemple comme agents tensioactifs siliconés les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination ABIL EM 90 par la société Goldschmidt, ou le mélange polyglycéryl-4 isostéarate/cétyl diméthicone copolyol/hexyllaurate vendu sous la dénomination ABIL WE 09 par la société Goldschmidt.

On peut y ajouter aussi un ou plusieurs co-émulsionnants. De manière avantageuse, le co-émulsionnant peut être choisi dans le groupe comprenant les esters alkylés de polyol. Comme esters alkylés de polyol, on peut citer notamment les esters de glycérol et/ou de sorbitan et par exemple le polyglyceryl-3 diisostearate commercialisé sous la dénomination de LAMEFORM TGI par la société Cognis, l'isostéarate de polyglycérol-4, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

On peut aussi utiliser comme agents tensioactifs d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Comme agent tensioactif adapté à l'obtention d'une émulsion E/H, conviennent notamment les agents tensioactifs polyisobutylene à terminaison succinique estérifiée, tels que ceux commercialisés sous les noms de Lubrizol 5603^{®} et Chemcinnate 2000^{®} par les sociétés Lubrizol et Chemron.
Conviennent également à l'invention, les agents tensioactifs de type polymères amphiphiles.

On entend par polymère amphiphile, tous polymères comportant à la fois une partie hydrophile et une partie hydrophobe et ayant la propriété de former un film séparant deux liquides de polarité différente et permettant ainsi de stabiliser des dispersions liquide - liquide de type direct, inverse ou multiple. Ces polymères peuvent être hydrosolubles ou hydrodispersibles.

On peut utiliser plus particulièrement les copolymères acrylate/C₁₀-C₃₀-alkylacrylate tels que les produits vendus sous les noms PEMULEN TR1, PEMULEN TR2 et CARBOBOL 1382 par la Société GOODRICH, ou bien leurs mélanges. On peut utiliser aussi les copolymères acrylate/steareth-20 itaconate et copolymères acrylate/ceteth-20 itaconate vendus sous les noms STRUCTURE 2001 et STRUCTURE 3001 par la Société NATIONAL STARCH. A titre de terpolymères pouvant être utilisés, on peut citer le terpolymère acide méthacrylique/acrylate de méthyle/diméthyl m-isopropénylbenzylisocyanate d'alcool béhénylique éthoxylé à 40 OE,(c'est-à-dire comportant 40 groupes oxyéthylénés) vendus par la société AMERCHOL sous les dénominations VISCOPHOBE DB 1000 NP3-NP4.

On peut également citer les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous la dénomination SALCARE SC 80.

On peut également citer les polymères anioniques comme par exemple les polymères d'acide isophtalique ou d'acide sulfoisophtalique, et en particulier les copolymères de phtalate/sulfoisophtalate/glycol (par exemple diéthylèneglycol/Phtalate/isophtalate/1,4-cyclohexane-diméthanol) vendus sous les dénominations « Eastman AQ polymer » (AQ35S, AQ38S, AQ55S, AQ48 Ultra) par la société Eastman Chemical.

Selon un mode de réalisation, une composition de l'invention peut avantageusement comprendre moins de 5 % en poids, voire moins de 3 % en poids, voire moins de 2 % en poids par rapport au poids total de la composition, voire être dépourvue de tensio actifs siliconés.

### MILIEU PHYSIOLOGIQUEMENT ACCEPTABLE

Outre, les composés indiqués précédemment, une composition selon l'invention comprend un milieu physiologiquement acceptable.

Par « milieu physiologiquement acceptable », on entend désigner un milieu convenant particulièrement à l'application d'une composition de l'invention sur les matières kératiniques, notamment la peau et les lèvres.

Le milieu physiologiquement acceptable est généralement adapté à la nature du support sur lequel doit être appliquée la composition, ainsi qu'à l'aspect sous lequel la composition doit être conditionnée.

### Phase aqueuse

Une composition de l'invention peut comprendre de l'eau en une teneur variant de 5 à 80 %, et plus particulièrement de 20 à 60 % en poids par rapport au poids total de la composition.

Une eau convenant à l'invention peut, par exemple, être une eau de source naturelle, telle que l'eau de La Roche-Posay ou une eau florale.

Selon un mode de réalisation, une composition de l'invention peut comprendre en outre au moins un solvant organique miscible à l'eau.

Le ou les solvant(s) organique(s) miscible(s) à l'eau convenant à l'invention peu(vent)t être choisi(s) parmi les monoalcools en C₁₋₈, et notamment en C₁₋₅, notamment l'éthanol, l'isopropanol, le tert-butanol, le n-butanol, les polyols, les glycols en C₂-C₈, les alcools polyhydriques en C₂-C₆, comme la glycérine et leurs mélanges.

Une composition de l'invention peut en outre comprendre au moins un sel, par exemple, le chlorure de sodium, le chlorure de magnésium et le sulfate de magnésium.

Une composition de l'invention peut comprendre de 0,1 % à 1,5 % en particulier de 0,4 % à 1,2 % et plus particulièrement de 0,5 % à 1 % en poids de sels, par rapport au poids total de la composition.

### Phase grasse liquide

Une composition cosmétique conforme à la présente invention peut comprendre, outre au moins une huile végétale comme indiqué précédemment, au moins une phase grasse liquide et/ou solide et notamment au moins une huile comme mentionnée ci-après.

On entend par huile, tout corps gras sous forme liquide à température ambiante (20 - 25 °C) et à pression atmosphérique.

Une composition de l'invention peut comprendre une phase grasse liquide en une teneur variant de 5 à 95 %, en particulier de 10 à 80 %, en particulier de 15 à 70 %, et plus particulièrement, de 20 à 65 % en poids par rapport au poids total de la composition.

La phase huileuse convenant à la préparation des compositions cosmétiques selon l'invention peut comprendre des huiles d'hydrocarbures, des huiles hydrocarbonées, siliconées, fluorées ou non, ou leurs mélanges.

Les huiles peuvent être volatiles ou non volatiles.

Elles peuvent être d'origine animale, végétale, minérale ou synthétique. Selon une variante de réalisation, les huiles d'origine végétale sont préférées.

Au sens de la présente invention, on entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et à pression atmosphérique, en particulier, ayant une pression de vapeur allant de 0,13 Pa à 40 000 Pa (10⁻³ à 300 mm Hg), et de préférence, allant de 1,3 Pa à 13 000 Pa (0,01 à 100 mm Hg), et préférentiellement allant de 1,3 Pa à 1300 Pa (0,01 à 10 mm Hg).

Au sens de la présente invention, on entend par « huile non volatile », une huile ayant une pression de vapeur inférieure à 0,13 Pa.

Au sens de la présente invention, on entend par « huile siliconée », une huile comprenant au moins un atome de silicium, et notamment au moins un groupe Si-O.

On entend par « huile fluorée », une huile comprenant au moins un atome de fluor.

On entend par « huile hydrocarbonée », une huile contenant principalement des atomes d'hydrogène et de carbone, et le cas échéant des fonctions alcool, acide, ester ou éther.

Une huile d'hydrocarbures est une huile hydrocarbonée composée exclusivement d'atomes d'hydrogène et de carbone.

Les huiles peuvent éventuellement comprendre des atomes d'oxygène, d'azote, de soufre et/ou de phosphore, par exemple, sous la forme de radicaux hydroxyle ou acide.

Une huile peut être une huile polymérique ou une huile non-polymérique. Par « huile polymérique », on entend une huile comprenant au moins une ou étant constituée exclusivement de molécules formées de la répétition de plusieurs monomères, identiques ou différents. A titre d'huile polymérique on peut mentionner le polyisobutène.

### Huiles volatiles

Les huiles volatiles peuvent être choisies parmi les huiles hydrocarbonées ayant de 8 à 16 atomes de carbone, et notamment les alcanes ramifiés en C₈-C₁₆ (appelées aussi isoparaffines), comme l'isododécane (encore appelé 2,2,4,4,6-pentaméthylheptane), l'isodécane, l'isohexadécane, et, par exemple, les huiles vendues sous les noms commerciaux d'ISOPARS^{®} ou de PERMETHYLS^{®}.

Comme huiles volatiles, on peut aussi utiliser les silicones volatiles, comme, par exemple, les huiles de silicones linéaires ou cycliques volatiles, notamment, celles ayant une viscosité ≤ 8 centistokes (cSt) (8 x 10⁻⁶ m²/s), et ayant, notamment, de 2 à 10 atomes de silicium, et en particulier, de 2 à 7 atomes de silicium, ces silicones comportant, éventuellement, des groupes alkyle ou alkoxy ayant de 1 à 10 atomes de carbone. Comme huile de silicone volatile utilisable dans l'invention, on peut citer, notamment, les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées, telles que le nonafluorométhoxybutane ou le perfluorométhylcyclopentane, et leurs mélanges.

### Huiles non volatiles

Les huiles non volatiles peuvent, notamment, être choisies parmi les huiles hydrocarbonées, fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale, telles que les esters de phytostéaryle, tels que l'oléate de phytostéaryle, l'isostéarate de physostéaryle et le glutamate de lauroyl/octyldodécyle/phytostéaryle (AJINOMOTO, ELDEW PS203), les triglycérides constitués d'esters d'acides gras et de glycérol, en particulier, dont les acides gras peuvent avoir des longueurs de chaînes variant de C₄ à C₃₆, et, notamment, de C₁₈ à C₃₆, ces huiles pouvant être linéaires ou ramifiées, saturées ou insaturées; ces huiles peuvent, notamment, être des triglycérides héptanoïques ou octanoïques, ou encore des triglycérides d'acides caprylique/caprique, comme ceux vendus par la société STEARINERIES DUBOIS ou ceux vendus sous les dénominations MIGLYOL 810^{®}, 812^{®} et 818^{®} par la société DYNAMIT NOBEL,
- les éthers de synthèse ayant de 10 à 40 atomes de carbone; comme le dicapryl ether.
- les esters de synthèse, comme les huiles de formule R₁COOR₂, dans laquelle R₁ représente un reste d'un acide gras linéaire ou ramifié comportant de 1 à 40 atomes de carbone et R₂ représente une chaîne hydrocarbonée, notamment, ramifiée contenant de 1 à 40 atomes de carbone à condition que R₁ + R₂ soit ≥ 10. Les esters peuvent être, notamment, choisis parmi les esters d'alcool et d'acide gras, comme par exemple :
   - l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate d'isopropyle, le stéarate d'octyle, les esters hydroxylés, comme le lactate d'isostéaryle, l'hydroxystéarate d'octyle, les ricinoléates d'alcools ou de polyalcools, le laurate d'hexyle, les esters de l'acide néopentanoïque, comme le néopentanoate d'isodécyle, le néopentanoate d'isotridécyle, les esters de l'acide isononanoïque, comme l'isononanoate d'isononyle, l'isononanoate d'isotridécyle,
- les esters de polyols et les esters du pentaérythritol, comme le tétrahydroxystéarate/tétraisostéarate de dipentaérythritol,
- les alcools gras liquides à température ambiante à chaîne carbonée ramifiée et/ou insaturée ayant de 12 à 26 atomes de carbone, comme le 2-octyldodécanol, l'alcool isostéarylique, l'alcool oléique,
- les acides gras supérieurs en C₁₂-C₂₂, tels que l'acide oléique, l'acide linoléique, l'acide linolénique et leurs mélanges, et
- les carbonates de di-alkyle, les 2 chaînes alkyles pouvant être identiques ou différentes, tels que le dicaprylyl carbonate commercialisé sous la dénomination CETIOL CC^{®}, par COGNIS,
- les huiles de masse molaire élevée ayant, en particulier, une masse molaire allant d'environ 400 à environ 2000 g/mol, en particulier, d'environ 650 à environ 1600 g/mol. Comme huile de masse molaire élevée utilisable dans la présente invention, on peut notamment citer les huiles choisies parmi :
   a) les esters, tels que :
      - les esters d'acides gras linéaires ayant un nombre total de carbone allant de 35 à 70, comme le tétrapélargonate de pentaérythrityle,
      - les esters hydroxylés, tels que le triisostéarate de polyglycérol-2,
      - les esters aromatiques, tels que le tridécyl trimellitate,
      - les esters d'alcools gras ou d'acides gras ramifiés en C₂₄-C₂₈, tels que ceux décrits dans le brevet US 6,491,927 et les esters du pentaérythritol, et, notamment, le citrate de triisoarachidyle, le triisostéarate de glycéryle, le tri décyl-2 tétradécanoate de glycéryle, le tétraisostéarate de polyglycéryle-2 ou encore le tétra décyl-2 tétradécanoate de pentaérythrityle,
   b) les huiles siliconées, telles que les silicones phénylées comme la BELSIL PDM 1000 de la société WACKER (MM=9000 g/mol). D'autres huiles de silicone non volatiles utilisables dans la composition selon l'invention peuvent être les polydiméthylsiloxanes (PDMS) non volatiles, les PDMS comportant des groupements alkyle ou alcoxy pendants et/ou en bouts de chaîne siliconée, groupements ayant chacun de 2 à 24 atomes de carbone, les silicones phénylées, comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 cSt, et leurs mélanges,
ainsi que les mélanges de ces différentes huiles.

Selon un mode de réalisation, une composition de l'invention peut avantageusement comprendre moins de 10 % en poids, voire moins de 5 % en poids, voire moins de 2 % en poids par rapport au poids total de la composition, voire être dépourvue d'huile siliconée, en particulier d'huile siliconée cyclique, et/ou d'huile minérale, et/ou d'alcanes volatils ramifiés n'étant pas directement issus de végétaux ou n'étant pas d'origine végétale, comme l'isododécane ou les isoparaffines.

Selon un autre mode de réalisation, une composition de l'invention peut comprendre au moins 20 % d'alcane linéaire volatil et/ou de solvant volatil de type alcane(s) linéaire(s) volatil(s) par rapport à la teneur totale en huile hydrocarbonée de la composition.

Selon un mode de réalisation, une composition de l'invention peut comprendre au moins 30 %, voire au moins 40 %, en particulier au moins 50 %, notamment au moins 60 %, plus particulièrement au moins 70 %, et plus particulièrement au moins 80 %, au moins 90 % ou 100 % d'alcane linéaire volatil et/ou de solvant volatil de type alcane(s) linéaire(s) volatil(s) par rapport à la teneur totale en huile hydrocarbonée de la composition.

Une composition de l'invention comprenant 100 % d'alcane linéaire volatil et/ou de solvant volatil de type alcane(s) linéaire(s) volatil(s) par rapport à la teneur totale en huile hydrocarbonée comprend une phase huileuse hydrocarbonée composée exclusivement d'alcane linéaire volatil et/ou de solvant volatil de type alcane(s) linéaire(s) volatil(s).

Selon une variante de réalisation, une composition de l'invention peut comprendre au moins 20 % d'alcane linéaire volatil et/ou de solvant volatil de type alcane(s) linéaire(s) volatil(s) par rapport à la teneur totale en huile d'hydrocarbures, en particulier par rapport à la teneur totale en huile d'hydrocarbures saturés, et de préférence par rapport à la teneur totale en huile d'hydrocarbures saturés non polymériques de la composition.

Selon un mode de réalisation, une composition de l'invention peut comprendre au moins 30 %, voire au moins 40 %, en particulier au moins 50 %, notamment au moins 60 %, plus particulièrement au moins 70 %, et plus particulièrement au moins 80 %, au moins 90 % ou 100 % d'alcane linéaire volatil et/ou de solvant volatil de type alcane(s) linéaire(s) volatil(s) par rapport à la teneur totale en huile d'hydrocarbures, en particulier par rapport à la teneur totale en huile d'hydrocarbures saturés, et de préférence par rapport à la teneur totale en huile d'hydrocarbures saturés non polymériques de la composition.

Selon une variante de réalisation, une huile d'hydrocarbures considérée dans les modes de réalisation exposés ci-dessus peut présenter une masse molaire inférieure à 650 g/mol, et ne particulier inférieure à 400 g/mol.

Une composition de l'invention comprenant 100% d'alcane linéaire volatil et/ou de solvant volatil de type alcane(s) linéaire(s) volatil(s) par rapport à la teneur totale en huile d'hydrocarbures ou en huile d'hydrocarbures saturés, ou en huile d'hydrocarbures saturés non polymériques comprend une phase huileuse d'hydrocarbures, d'hydrocarbures saturés, ou d'hydrocarbures saturés non polymérique composée exclusivement d'alcane linéaire volatil et/ou de solvant volatil de type alcane(s) linéaire(s) volatil(s).

Selon un autre mode de réalisation, une composition de l'invention peut comprendre moins de 5 % en poids, de préférence moins de 2 % en poids d'huile d'hydrocarbures ramifiés et/ou cycliques par rapport au poids total de la composition.

### Agent structurant lipophile

Une composition selon l'invention peut comprendre au moins un agent structurant de phase grasse liquide choisi parmi une cire, un composé pâteux et leurs mélanges.

Une composition selon l'invention peut comprendre de 0,1 à 20 % en poids d'agent structurant de phase grasse liquide par rapport au poids total de la composition, en particulier de 0,3 à 15 % d'agent structurant de phase grasse liquide, et plus particulièrement de 0,5 à 10 % en poids d'agent structurant de phase grasse liquide par rapport au poids total de la composition.

### Cire(s)

Une composition de l'invention peut comprendre au moins une cire.

Une cire convenant à l'invention est, d'une manière générale, un composé lipophile, solide à température ambiante (25 °C), ayant un point de fusion supérieur ou égal à 30 °C, pouvant aller jusqu'à 200 °C et notamment jusqu'à 120 °C.

En portant une cire à l'état liquide (fusion), il est possible de la rendre miscible aux huiles et de former un mélange homogène macroscopiquement, mais en ramenant la température du mélange à la température ambiante, on obtient une recristallisation de la cire dans les huiles du mélange.

Une cire convenant à l'invention peut présenter un point de fusion supérieur ou égal à 45 °C, et en particulier, supérieur ou égal à 55 °C.

Au sens de l'invention, la température de fusion correspond à la température du pic le plus endothermique observé en analyse thermique (DSC), telle que décrite dans la norme ISO 11357-3 ; 1999. Le point de fusion de la cire peut être mesuré à l'aide d'un calorimètre à balayage différentiel (DSC), par exemple, le calorimètre vendu sous la dénomination « MDSC 2920 » par la société TA Instruments.

Une cire susceptible d'être utilisée dans une composition de l'invention peut être choisie parmi des cires solides à température ambiante.

En particulier, une cire convenant à l'invention peut être choisie parmi des cires d'origine animale, végétale, minérale, synthétique, et leurs mélanges.

Selon une variante de réalisation, les cires d'origine végétale sont préférées.

A titre illustratif de cires convenant à l'invention, on peut notamment citer les cires hydrocarbonées, comme la cire d'abeille, la cire de lanoline, et les cires d'insectes de Chine; la cire de son de riz, la cire de Carnauba, la cire de Candellila, la cire d'Ouricury, la cire d'Alfa, la cire de Berry, la cire de Shellac, la cire du Japon et la cire de sumac, la cire de Montan, les cires d'orange et de citron, les cires microcristallines, les paraffines et l'ozokérite; les cires de polyéthylène, les cires obtenues par la synthèse de Fisher-Tropsch et les copolymères cireux, ainsi que leurs esters.

On peut également citer les cires microcristallines en C₂₀-C₆₀, telles que la Microwax HW.

On peut également citer la cire de polyéthylène PM 500 commercialisée sous la référence Permalen 50-L polyéthylène.

On peut aussi citer des cires obtenues par hydrogénation catalytique d'huiles animales ou végétales ayant des chaînes grasses, linéaires ou ramifiées, en C₈-C₃₂.

Parmi celles-ci, on peut notamment citer l'huile de jojoba isomérisée, telle que l'huile de jojoba partiellement hydrogénée isomérisée trans fabriquée ou commercialisée par la société DESERT WHALE sous la référence commerciale Iso-Jojoba-50^{®}, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S^{®} par la société HETERENE.

On peut encore citer les cires de silicone (C₃₀₋₄₅ ALKYL DIMETHICONE) et les cires fluorées.

On peut également utiliser les cires obtenues par hydrogénation d'huile de ricin estérifiée avec l'alcool cétylique vendues sous les dénominations de Phytowax ricin 16L64^{®} et 22L73^{®} par la société SOPHIM. De telles cires sont décrites dans la demande FR-A- 2792190.

Comme cire, on peut utiliser un (hydroxystéaryloxy)stéarate d'alkyle en C₂₀-C₄₀ (le groupe alkyle comprenant de 20 à 40 atomes de carbone), seul ou en mélange.

Une telle cire est notamment vendue sous les dénominations « Kester Wax K 82 P^{®} », « Hydroxypolyester K 82 P^{®} » et « Kester Wax K 80 P^{®} » par la société KOSTER KEUNEN.

Selon un mode de réalisation, une composition de l'invention peut avantageusement comprendre moins de 10 % en poids par rapport au poids total de la composition de cires siliconées et/ou de cires minérales, voire moins de 5 % en poids, voire moins de 2 % en poids de cires siliconées et/ou de cires minérales par rapport au poids total de la composition, voire être dépourvue de cires siliconées et/ou de cires minérales.

### Composés pâteux

Une composition selon l'invention peut comprendre au moins un composé pâteux. La présence d'un composé pâteux peut permettre de conférer avantageusement un confort amélioré lors du dépôt d'une composition de l'invention sur les matières kératiniques.

Un tel composé peut être avantageusement choisi parmi :
- la lanoline et ses dérivés,
- les composés siliconés polymériques ou non,
- les composés fluorés polymériques ou non,
- les polymères vinyliques, notamment :
   - les homopolymères d'oléfines,
   - les copolymères d'oléfines,
   - les homopolymères et copolymères de diènes hydrogénés,
   - les oligomères linéaires ou ramifiés, homo ou copolymères de (méth)acrylates d'alkyles ayant de préférence un groupement alkyle en C₈-C₃₀,
   - les oligomères homo et copolymères d'esters vinyliques ayant des groupements alkyle en C₈-C₃₀,
   - les oligomères homo et copolymères de vinyléthers ayant des groupements alkyle en C₈-C₃₀,
- les polyéthers liposolubles résultant de la polyéthérification entre un ou plusieurs diols en C₂-C₁₀₀, en particulier, en C₂-C₅₀,
- les esters d'acide ou d'alcool gras,
- et leurs mélanges.

Parmi les esters, on peut notamment citer :
- les esters d'un glycérol oligomère, notamment, les esters de diglycérol, en particulier les condensats d'acide adipique et de glycérol, pour lesquels une partie des groupes hydroxyle des glycérols ont réagi avec un mélange d'acides gras, tels que l'acide stéarique, l'acide caprique, l'acide stéarique et isostéarique et l'acide 12-hydroxystéarique, à l'image, notamment, de ceux commercialisé sous la marque Softisan 649 par la société Sasol ou tel que le polyacyladipate-2 de bis diglycéryle,
- le propionate d'arachidyle commercialisé sous la marque Waxenol 801 par Alzo,
- les esters de phytostérol,
- les triglycérides d'acides gras et leurs dérivés, tels que les coco-glycérides hydrogénés,
- les polyesters non réticulés résultant de la polycondensation entre un acide dicarboxylique ou un polyacide carboxylique linéaire ou ramifié en C₄-C₅₀ et un diol ou un polyol en C₂-C₅₀,
- les esters aliphatiques d'ester résultant de l'estérification d'un ester d'acide hydroxycarboxylique aliphatique par un acide carboxylique aliphatique,
- et leurs mélanges.

Selon un mode de réalisation, une composition de l'invention peut avantageusement comprendre moins de 10 % en poids par rapport au poids total de la composition de pâteux siliconés et/ou de pâteux fluorés, voire moins de 5 % en poids de pâteux siliconés et/ou de pâteux fluorés, voire moins de 2 % en poids par rapport au poids total de la composition, voire être dépourvue de pâteux siliconés et/ou de pâteux fluorés.

### GELIFIANTS

Selon la fluidité de la composition que l'on souhaite obtenir, on peut incorporer dans une composition de l'invention, un ou plusieurs gélifiants, notamment hydrophiles, c'est-à-dire solubles ou dispersibles dans l'eau.

Comme gélifiants hydrophiles, on peut citer en particulier les polymères épaississants hydrosolubles ou hydrodispersibles. Ceux-ci peuvent notamment être choisis parmi : les polymères carboxyvinyliques modifiés ou non, tels que les produits commercialisés sous les dénominations Carbopol (nom CTFA : carbomer) par la société Goodrich ; les polyacrylates et polyméthacrylates tels que les produits vendus sous les dénominations de Lubrajel et Norgel par la société GUARDIAN ou sous la dénomination Hispagel par la société HISPANO CHIMICA ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom C.T.F.A. : Polyacrylamide / C13-14 Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les biopolymères polysaccharidiques comme la gomme de xanthane, la gomme de guar, la gomme de caroube, la gomme d'acacia, les scléroglucanes, les dérivés de chitine et de chitosane, les carraghénanes, les gellanes, les alginates, les celluloses telles que la cellulose microcristalline, la carboxyméthylcellulose, l'hydroxyméthylcellullose et l'hydroxypropylcellulose ; et leurs mélanges.

Comme gélifiants lipophiles, on peut citer par exemple les argiles modifiées telles que le silicate de magnésium modifié (Bentone gel VS38 de RHEOX), l'hectorite modifiée par le chlorure de distéaryl diméthyl ammonium (nom CTFA : Disteardimonium hectorite) commercialisé sous la dénomination « Bentone 38 CE » par la société RHEOX.

### MATIERES COLORANTES

Une composition selon l'invention peut en outre comprendre au moins une matière colorante.

Une composition cosmétique conforme à l'invention peut, avantageusement, incorporer au moins une matière colorante choisie parmi des matières colorantes organiques ou inorganiques, notamment de type pigments ou nacres classiquement utilisés dans les compositions cosmétiques, liposolubles ou hydrosolubles, des matériaux à effet optique spécifique, et leurs mélanges.

Avantageusement, une composition de l'invention peut comprendre au moins un pigment.

Par pigments, il faut comprendre des particules blanches ou colorées, minérales ou organiques, insolubles dans une solution aqueuse, destinées à colorer et/ou opacifier le film résultant.

Les pigments peuvent être présents à raison de 0,01 à 40 % en poids, notamment, de 0,1 à 20 % en poids, et en particulier, de 1 à 15 % en poids, par rapport au poids total de la composition cosmétique.

Comme pigments minéraux utilisables dans l'invention, on peut citer les oxydes de titane, de zirconium ou de cérium, ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

La matière colorante peut encore comporter un pigment ayant une structure qui peut être, par exemple, de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Par « nacres », il faut comprendre des particules colorées de toute forme, irisées ou non, notamment, produites par certains mollusques dans leur coquille ou bien synthétisées et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA, FLAMENCO et DUOCHROME (sur base de mica) commercialisées par la société ENGELHARD, les nacres TIMIRON commercialisées par la société MERCK, les nacres sur base de mica PRESTIGE commercialisées par la société ECKART et les nacres sur base de mica synthétique SUNSHINE commercialisées par la société SUN CHEMICAL.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut, notamment, citer les nacres de couleur or, notamment, commercialisées par la société ENGELHARD, sous le nom de Brillant gold 212G (Timica), Gold 222C (Cloisonne), Sparkle gold (Timica), Gold 4504 (Chromalite) et Monarch gold 233X (Cloisonne) ; les nacres bronzes, notamment, commercialisées par la société MERCK sous la dénomination Bronze fine (17384) (Colorona) et Bronze (17353) (Colorona) et par la société ENGELHARD sous la dénomination Super bronze (Cloisonne); les nacres oranges, notamment, commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune, notamment, commercialisées par la société ENGELHARD sous la dénomination Nu-antique copper 340XB (Cloisonne) et Brown CL4509 (Chromalite) ; les nacres à reflet cuivre, notamment, commercialisées par la société ENGELHARD sous la dénomination Copper 340A (Timica) ; les nacres à reflet rouge, notamment, commercialisées par la société MERCK sous la dénomination Sienna fine (17386) (Colorona) ; les nacres à reflet jaune, notamment, commercialisées par la société ENGELHARD sous la dénomination Yellow (4502) (Chromalite) ; les nacres de teinte rouge à reflet or, notamment, commercialisées par la société ENGELHARD sous la dénomination Sunstone G012 (Gemtone) ; les nacres roses, notamment, commercialisées par la société ENGELHARD sous la dénomination Tan opale G005 (Gemtone) ; les nacres noires à reflet or, notamment, commercialisées par la société ENGELHARD sous la dénomination Nu antique bronze 240 AB (Timica), les nacres bleues, notamment, commercialisées par la société MERCK sous la dénomination Matte blue (17433) (Microna), les nacres blanches à reflet argenté, notamment, commercialisées par la société MERCK sous la dénomination Xirona Silver et les nacres orangées rosées vert doré, notamment, commercialisées par la société MERCK sous la dénomination Indian summer (Xirona) et leurs mélanges.

La composition cosmétique selon l'invention peut comprendre également des colorants hydrosolubles ou liposolubles. Les colorants liposolubles sont, par exemple, le rouge Soudan, le DC Red 17, le DC Green 6, le β-carotène, le brun Soudan, le DC Yellow 11, le DC Violet 2, le DC orange 5 et le jaune quinoléine. Les colorants hydrosolubles sont, par exemple, le jus de betterave et le caramel.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques, comme, par exemple, les pigments monochromatiques. Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment, interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :
- les particules d'au moins un métal et/ou d'au moins un dérivé métallique,
- les particules comportant un substrat organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple, les bronzes et les laitons) sont des métaux préférés.

Par « dérivés métalliques », on désigne des composés dérivés de métaux, notamment, des oxydes, des fluorures, des chlorures et des sulfures

A titre illustratif de ces particules, on peut citer des particules d'aluminium, telles que celles commercialisées sous les dénominations STARBRITE 1200 EAC^{®} par la société SIBERLINE et METALURE^{®} par la société ECKART.

On peut également citer les poudres métalliques de cuivre ou des mélanges d'alliages, telles les références 2844 commercialisées par la société RADIUM BRONZE, les pigments métalliques, comme l'aluminium ou le bronze, tels que ceux commercialisés sous les dénominations ROTOSAFE 700 de la société ECKART, les particules d'aluminium enrobé de silice commercialisées sous la dénomination VISIONAIRE BRIGHT SILVER de la société ECKART et les particules d'alliage métallique, comme des poudres de bronze (alliage cuivre et zinc) enrobé de silice commercialisées sous la dénomination de Visionaire Bright Natural Gold de la société Eckart.

Il peut encore s'agir de particules comportant un substrat de verre comme celles commercialisées par la société NIPPON SHEET GLASS sous les dénominations MICROGLASS METASHINE.

L'agent de coloration goniochromatique peut être choisi, par exemple, parmi les structures multicouches interférentielles et les agents de coloration à cristaux liquides.

Des exemples de structures multicouche interférentielles symétriques utilisables dans des compositions réalisées conformément à l'invention sont, par exemple, les structures suivantes : Al/SiO₂/Al/SiO₂/Al, des pigments ayant cette structure étant commercialisés par la société DUPONT DE NEMOURS; Cr/MgF₂/Al/MgF₂/Cr, des pigments ayant cette structure étant commercialisés sous la dénomination CHROMAFLAIR par la société FLEX ; MoS₂/SiO₂/Al/SiO₂/MoS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination SICOPEARL par la société BASF ; MoS₂/SiO₂/mica-oxyde/SiO₂/MoS₂; Fe₂O₃/SiO₂/mica-oxyde/SiO₂/Fe₂O₃ TiO₂/SiO₂/TiO₂ et TiO₂/Al₂O₃/TiO₂ ; SnO/TiO₂/SiO₂/TiO₂/SnO ; Fe₂O₃/SiO₂/Fe₂O₃ ; SnO/mica/TiO₂/SiO₂/TiO₂/mica/SnO, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut citer, à titre d'exemple de pigments à structure multicouche polymérique, ceux commercialisés par la société 3M sous la dénomination COLOR GLITTER.

Comme particules goniochromatiques à cristaux liquides, on peut utiliser, par exemple, celles vendues par la société CHENIX, ainsi que celle commercialisées sous la dénomination HELICONE^{®} HC par la société WACKER.

### CHARGES

Une composition conforme à l'invention peut également comprendre outre du carbonate de calcium, au moins une charge, de nature organique ou minérale.

Par « charge », il faut comprendre les particules incolores ou blanches, solides de toutes formes, qui se présentent sous une forme insoluble et dispersée dans le milieu de la composition. De nature minérale ou organique, elles permettent de conférer à la composition de la douceur, de la matité et de l'uniformité au maquillage.

Les charges utilisées dans les compositions selon la présente invention peuvent être de formes lamellaires, globulaires, sphériques, de fibres ou de toute autre forme intermédiaire entre ces formes définies.

Les charges selon l'invention peuvent être ou non enrobées superficiellement, et, en particulier, elles peuvent être traitées en surface par des silicones, des acides aminés, des dérivés fluorés ou toute autre substance favorisant la dispersion et la compatibilité de la charge dans la composition.

Au sens de la présente invention, les termes « charges minérales » et « charges inorganiques » sont utilisés de manière interchangeable.

Parmi les charges minérales utilisables dans les compositions selon l'invention, on peut citer le talc, le mica, la silice, le siloxysilicate de triméthyle, le kaolin, la bentone, le carbonate et l'hydrogéno-carbonate de magnésium, l'hydroxyapatite, le nitrure de bore, les microsphères de silice creuses (Silice Beads de Maprecos), les microcapsules de verre ou de céramique, les charges à base de silice comme l'Aerosil 200, l'Aerosil 300 ; la Sunsphere H-33, la Sunsphere H-51 commercialisés par Asahi Glass ; le Chemicelen commercialisé par Asahi Chemical ; les composites de silice et de dioxyde de titane, comme la série TSG commercialisée par Nippon Sheet Glass, et leurs mélanges.

Parmi les charges organiques utilisables dans les compositions selon l'invention on peut citer les poudres de polyamide (Nylon^{®} Orgasol de chez Atochem), de poly-b-alanine et polyéthylène, les poudres de polytétrafluoroéthylène (Téflon^{®}), la lauroylysine, l'amidon, les poudres de polymères de tétrafluoroéthylène, les microsphères creuses de polymères, telles l'EXPANCEL (NOBEL INDUSTRIE), les savons métalliques dérivés d'acides organiques carboxyliques ayant de 8 à 22 atomes de carbone, de préférence, de 12 à 18 atomes de carbone, par exemple, le stéarate de zinc, de magnésium ou de lithium, le laurate de zinc, le myristate de magnésium, le Polypore® L 200 (Chemdal Corporation), les microbilles de résine de silicone (Tospearl ® de Toshiba, par exemple), les poudres de polyuréthane, en particulier, les poudres de polyuréthane réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple, sous la dénomination de PLASTIC POWDER D-400^{®} ou PLASTIC POWDER D-800^{®} de la société TOSHIKI, les microcires de Carnauba, telles que celle commercialisée sous la dénomination de MicroCare 350^{®} par la société MICRO POWDERS, les microcires de cire synthétique, telles que celle commercialisée sous la dénomination de MicroEase 114S^{®} par la société MICRO POWDERS, les microcires constituées d'un mélange de cire de Carnauba et de cire de polyéthylène, telles que celles commercialisées sous les dénominations de Micro Care 300^{®} et 310^{®} par la société MICRO POWDERS, les microcires constituées d'un mélange de cire de Carnauba et de cire synthétique, telles que celle commercialisée sous la dénomination Micro Care 325^{®} par la société MICRO POWDERS, les microcires de polyéthylène, telles que celles commercialisées sous les dénominations de Micropoly 200^{®}, 220^{®}, 220L^{®} et 250S^{®} par la société MICRO POWDERS et les microcires de polytétrafluoroéthylène, telles que celles commercialisées sous les dénominations de Microslip 519^{®} et 519 L^{®} par la société MICRO POWDERS. et leurs mélanges.

Les microcires naturelles ou d'origine naturelle sont préférées, telles que les microcires de Carnauba.

### ACTIFS

Une composition de l'invention peut comprendre en outre au moins un actif cosmétique et/ou un actif dermatologique.

A titre d'exemples, non limitatifs, d'actifs cosmétiques et/ou dermatologiques convenant à l'invention ou peut citer les actifs choisis parmi les agents suivants :
les agents hydratants, les agents desquamants, les agents améliorant la fonction barrière, les agents dépigmentants, les agents antioxydants, les agents dermodécontractants, les agents anti-glycation, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisant la maturation de l'enveloppe cornée, les inhibiteurs de NO-synthases, les antagonistes des récepteurs périphériques des benzodiazépines (PBR), les agents augmentant l'activité de la glande sébacée, les agents stimulant le métabolisme énergétique des cellules, les agents tenseurs, les agents liporestructurants, les agents amincissants, les agents favorisant la microcirculation cutanée, les agents apaisants et/ou anti-irritants, les sébo-régulateurs ou anti-séborrhéiques, les agents astringents, les agents cicatrisants, les agents anti-inflammatoires, les agents anti-acné, les agents matifiants, les charges à effet flouteur, les agents fluorescents, les agents favorisant la coloration naturellement rosée de la peau, les charges abrasives ou exfoliantes, les agents anti-microbiens, et les agents apaisants, les agents anti-NO, les agents anti-radicalaires ou anti-pollution, les filtres solaires, les vitamines telles que le tocophérol, et leurs mélanges.

Les actifs cosmétiques et/ou dermatologiques convenant à l'invention peuvent notamment être choisis parmi les agents mentionnés dans la demande EP 1 847 247.

Selon un mode de réalisation, une composition de l'invention destinée à la protection solaire des matières kératiniques, et notamment de la peau du corps ou des lèvres, peut en particulier comprendre à titre de filtres solaires les dérivés cinnamiques, des dérivés salicyliques, des dérivés du camphre, des dérivés de triazine, des dérivés de la benzophénone, des dérivés du dibenzoleméthane, des dérivés du β-diphénylacrylate, des dérivés de l'acide p-aminobenzoïque, des polymères filtres et silicones filtres, tels que décrits par exemple dans la demande WO 93/04665.

Egalement, une composition de l'invention peut contenir des agents de bronzage et/ou de brunissage artificiels de la peau, tels que par exemple de la dihydroxyacétone (DHA).

Il relève des attributions de l'homme du métier de sélectionner le ou lesdits actifs et leur teneur en fonction de l'effet recherché sur les matières kératiniques, et de façon à ne pas affecter les propriétés cosmétiques des compositions de l'invention.

### ADDITIFS

Une composition cosmétique selon l'invention peut également comprendre en outre tout additif usuellement utilisé dans le domaine concerné, par exemple choisi parmi des agents filmogènes, et le cas échéant, des auxiliaires de filmification, des gommes, des polymères semi-cristallins, des agent antioxydants, des huiles essentielles, des conservateurs, des parfums, des neutralisants, des agents antiseptiques, des agents protecteurs contre les UV, et leurs mélanges.

Il relève des opérations de routine de l'homme de l'art d'ajuster la nature et la quantité des additifs présents dans les compositions conformes à l'invention, de telle sorte que les propriétés cosmétiques et les propriétés de stabilité désirées de celles-ci n'en soient pas affectées.

Une composition selon l'invention peut, notamment, se présenter sous la forme d'une composition de maquillage et/ou de soins de la peau ou des lèvres, en particulier, un rouge à lèvres, un baume à lèvres.

Selon un mode de réalisation, une composition de l'invention peut avantageusement se présenter sous la forme d'un fond de teint.

Une composition de l'invention peut être obtenue par tout procédé de préparation connu de l'homme de l'art.

La présente invention sera mieux comprise au moyen des exemples qui suivent.

Ceux-ci ne sont présentés qu'à titre d'illustration de l'invention et ne doivent pas être interprétés comme limitant la portée de celle-ci.

### EXEMPLES

### Exemples 1-3

Les trois exemples de fond de teint qui suivent montrent l'influence des alcanes linéaires volatils de l'invention (Ex.2) et du carbonate de calcium (Ex.3) sur la stabilité de la composition.

| Composés / références commerciales | | Ex.1 (Invention) | Ex.2 (Comparatif) | Ex.3 (Comparatif) |
|---|---|---|---|---|
| **A1** | Di capryl ether vendu sous la référence CETIOL OE par la Société COGNIS | 9,00 | 19,00 | 10,70 |
| **A2** | Hectorite modifiée vendue sous la référence BENTONE 38 VCG par la société ELEMENTIS | 0,85 | 0,85 | 0,85 |
| **A3** | PEG-30 Dipolyhydroxy stearate vendu sous la référence ARLACEL P 135 par la Société UNIQEMA | 5,00 | 5,00 | 5,00 |
| **A4** | Di capryl ether vendu sous la référence CETIOL OE par la Société COGNIS | 4,00 | 4,00 | 4,00 |
| | Oxyde de fer j aune enrobé de stéaroyl glutamate d'aluminium | 2,16 | 2,16 | 2,16 |
| | Oxyde de fer rouge enrobé de stéaroyl glutamate d'aluminium | 0,59 | 0,59 | 0,59 |
| | Oxyde de fer noir enrobé de stéaroyl glutamate d'aluminium | 0,25 | 0,25 | 0,25 |
| | Dioxyde de titane enrobé de stéaroyl glutamate d'aluminium | 9,00 | 9,00 | 9,00 |
| **A5** | Huile de Macadamia | 4,00 | 4,00 | 4,00 |
| | Mélange de tocophérols naturels avec de l'huile de soja (50/50) | 0,50 | 0,50 | 0,50 |
| **A6** | Undécane+Tridécane vendu sous la référence CETIOL UT par la société COGNIS | 10,00 | 0 | 10,00 |
| **A7** | Carbonate de calcium naturel vendu sous la référence OMYAPURE 35 LM-OG par la société OMYA | 4,00 | 4,00 | 0 |
| **B** | Eau déminéralisée | 37,05 | 37,05 | 39,35 |
| | Glycérol | 5,00 | 5,00 | 5,00 |
| | Chlorure de sodium | 0,60 | 0,60 | 0,60 |
| | Sorbitol en solution aqueuse à 70 % vendu sous la référence NEOSORB 70/70 B par la société ROQUETTE | 2,00 | 2,00 | 2,00 |
| | Alcool benzylique | 1,00 | 1,00 | 1,00 |
| **C** | Ethanol | 5,00 | 5,00 | 5,00 |
| | TOTAL | 100 % massique | 100 % massique | 100 % massique |

### Mode opératoire

Le constituant de la phase A1 est pesé dans un bécher principal, et placé ensuite sur une plaque magnétique chauffante, sous agitation (barreau aimanté, 250 trs.mn-1) et sous chauffage (90 °C).

Les phases A2 et A3 sont ajoutées en maintenant l'agitation et le chauffage jusqu'à l'obtention d'un mélange homogène.

La phase A4 est préparée séparément en broyant le mélange (pigments-Dicapryl ether) à la tri-cylindre, puis est ajoutée sous agitation au Mortiz (1500 trs.mn-1), dans le bécher principal.

Enfin, sont introduites les phases A5, A6 et A7.

La phase aqueuse B est préparée par addition de l'eau chauffée à 95 °C au glycérol. On incorpore le chlorure de sodium et le sorbitol. L'alcool benzylique est ensuite introduit à une température inférieure à 40 °C.

L'émulsion se fait à température ambiante : la phase aqueuse est versée dans la phase grasse en augmentant progressivement la vitesse d'agitation jusqu'à 4500 trs.mn-1. L'agitation est maintenue pendant 10 minutes.

La phase C est ajoutée en maintenant l'agitation jusqu'à son incorporation totale.

### Mesure de la stabilité

On mesure la stabilité de l'émulsion à l'aide d'une étuve à cycles comme indiqué précédemment.

Plus précisément, le fond de teint est mis dans un pilulier étanche de 30 ml qui est ensuite placé dans une étuve à cycles de marque VÖTCH VT4004.

L'étuve est programmée pour effectuer des cycles successifs de -20 °C à + 20 °C. Un cycle dure 24 heures et se décompose de la façon suivante : 6 heures à une température de 20 °C, puis 6 heures de descente de température jusqu'à -20 °C, puis 6 heures à une température de -20 °C et enfin 6 heures de remontée de température jusqu'à +20 °C.

Après chaque cycle, les aspects macroscopique et microscopique du produit sont évalués.

### Résultats

Les résultats de stabilité, correspondant, respectivement aux formulations des exemples 1, 2 et 3 et sont reportés dans le tableau I suivant :

**Tableau I**

| | Ex 1 (Invention) | Ex 2 (Comparatif) | Ex 3 (Comparatif) |
|---|---|---|---|
| Stabilité, aspect microscopique. | Emulsion stable après 10 cycles. Quelques gouttes éparses. | Emulsion instable dès le 4^{ème} cycle. | Emulsion instable après 10 cycles. Début de coalescence. |

Les résultats révèlent que l'émulsion selon l'invention présente une stabilité améliorée par rapport aux autres émulsions.

### Exemple 4

L'exemple de fond de teint qui suit permet de montrer l'influence de la nature de la charge et la supériorité du carbonate de calcium (Ex.1 précédent) sur la stabilité de la composition.

| Composés / références commerciales | | % massique |
|---|---|---|
| **A1** | Di capryl ether vendu sous la référence CETIOL OE par la Société COGNIS | 9,00 |
| **A2** | Hectorite modifiée vendue sous la référence BENTONE 38 VCG par la société ELEMENTIS | 0,85 |
| **A3** | PEG-30 Dipolyhydroxy stearate vendu sous la référence ARLACEL P 135 par la Société UNIQEMA | 5,00 |
| **A4** | Di capryl ether vendu sous la référence CETIOL OE par la Société COGNIS | 4,00 |
| | Oxyde de fer j aune enrobé de stéaroyl glutamate d'aluminium | 2,16 |
| | Oxyde de fer rouge enrobé de stéaroyl glutamate d'aluminium | 0,59 |
| | Oxyde de fer noir enrobé de stéaroyl glutamate d'aluminium | 0,25 |
| | Dioxyde de titane enrobé de stéaroyl glutamate d'aluminium | 9,00 |
| **A5** | Huile de Macadamia | 4,00 |
| | Mélange de tocophérols naturels avec de l'huile de soja (50/50) | 0,50 |
| **A6** | Undécane+Tridécane vendu sous la référence CETIOL UT par la société COGNIS | 10,00 |
| **A7** | Charge | 4,00 |
| **B** | Eau déminéralisée | 37,05 |
| | Glycérol | 5,00 |
| | Chlorure de sodium | 0,60 |
| | Sorbitol en solution aqueuse à 70 % vendu sous la référence NEOSORB 70/70 B par la société ROQUETTE | 2,00 |
| | Alcool benzylique | 1,00 |
| **C** | Ethanol | 5,00 |
| | TOTAL | 100 % |

| Nature de la charge | |
|---|---|
| Ex 1 (Invention) | Ex 4 (hors invention) |
| Carbonate de calcium (OMYAPURE 35 LM-OG, OMYA) | Lauroyl Lysine (AMIHOPE LL, AJINOMOTO) |

### Mode opératoire

Les compositions sont préparées comme indiqué précédemment.

### Mesure de la stabilité

La stabilité des émulsions est mesurée à l'aide d'une étuve à cycles comme décrit précédemment.

### Résultats

Les résultats de stabilité correspondant, respectivement, aux exemples 1 et 4, sont reportés dans le tableau suivant :

| | Ex 1 (Invention) | Ex 4 (Comparatif) |
|---|---|---|
| Stabilité, aspect microscopique. | Emulsion stable après 10 cycles. Quelques gouttes éparses. | Emulsion instable après 3 cycles Emulsion grossière avec lâchages. |

Les résultats indiquent que l'émulsion de l'invention présente une stabilité améliorée.

### Exemple 5:

### Fond de teint-émulsion E/H

| Composés/références commerciales | | % massique |
|---|---|---|
| **A1** | Dicapryl ether vendu sous la référence CETIOL OE par la Société COGNIS | 2,90 |
| **A2** | Hectorite modifiée vendue sous la référence BENTONE 38 VCG par la société ELEMENTIS | 1,20 |
| **A3** | PEG-30 Dipolyhydroxy stearate vendu sous la référence ARLACEL P 135 par la Société UNIQEMA | 5,00 |
| **A4** | Di capryl ether vendu sous la référence CETIOL OE par la Société COGNIS | 5,00 |
| | Oxyde de fer jaune enrobé de stéaroyl glutamate d'aluminium | 2,52 |
| | Oxyde de fer rouge enrobé de stéaroyl glutamate d'aluminium | 0,69 |
| | Oxyde de fer noir enrobé de stéaroyl glutamate d'aluminium | 0,29 |
| | Dioxyde de titane enrobé de stéaroyl glutamate d'aluminium | 10,50 |
| **A5** | Huile de Macadamia | 4,00 |
| | Mélange de tocophérols naturels avec de l'huile de soja (50/50) | 0,50 |
| **A6** | Undécane+Tridécane vendu sous la référence CETIOL UT par la société COGNIS | 13,00 |
| **A7** | Carbonate de calcium naturel vendu sous la référence OMYAPURE 35 LM-OG par la société OMYA | 4,00 |
| **B** | Eau déminéralisée | 32,80 |
| | Glycérol | 5,00 |
| | Chlorure de sodium | 0,60 |
| | Sorbitol en solution aqueuse à 70 % vendu sous la référence NEOSORB 70/70 B par la société ROQUETTE | 2,00 |
| | Alcool benzylique | 1,00 |
| **C** | Silice vendue sous la référence SHERON P1500 par la société CCIC | 4,00 |
| **D** | Ethanol | 5,00 |
| | TOTAL | 100 % |

### Mode opératoire

Le constituant de la phase A1 est pesé dans un bécher principal et placé ensuite sur une plaque magnétique chauffante, sous agitation (barreau aimanté, 250 trs.mn-1) et sous chauffage (90 °C).

Les phases A2 et A3 sont ajoutées et l'agitation et le chauffage sont maintenus jusqu'à l'obtention d'un mélange homogène.

La phase A4 est préparée séparément en broyant le mélange (pigments-Dicapryl ether) à la tri-cylindre puis, sous agitation au Mortiz (1500 trs.mn-1), dans le bécher principal. Ensuite, sont ajoutées aux constituants présents les phases A4, A5, A6 et enfin A7.

La phase aqueuse B est préparée par ajout de l'eau chauffée à 95 °C au glycérol et incorporation du chlorure de sodium et du sorbitol. L'alcool benzylique est ensuite incorporé à une température inférieure à 40 °C.

L'émulsion se fait à température ambiante : la phase aqueuse est versée dans la phase grasse en augmentant progressivement la vitesse d'agitation jusqu'à 4500 trs.mn-l.

Cette agitation est maintenue pendant 10 minutes.

Puis, on ajoute la phase C et enfin la phase D : on maintient l'agitation jusqu'à incorporation totale de D.

### Exemple 6:

### Fond de teint-émulsion E/H

| Composés/références commerciales | | % massique |
|---|---|---|
| **A1** | Dicapryl ether vendu sous la référence CETIOL OE par la Société COGNIS | 2,90 |
| **A2** | Hectorite modifiée vendue sous la référence BENTONE 38 VCG par la société ELEMENTIS | 1,20 |
| **A3** | PEG-30 Dipolyhydroxy stearate vendu sous la référence ARLACEL P 135 par la Société UNIQEMA | 5,00 |
| **A4** | Di capryl ether vendu sous la référence CETIOL OE par la Société COGNIS | 5,00 |
| | Oxyde de fer jaune enrobé de stéaroyl glutamate d'aluminium | 2,52 |
| | Oxyde de fer rouge enrobé de stéaroyl glutamate d'aluminium | 0,69 |
| | Oxyde de fer noir enrobé de stéaroyl glutamate d'aluminium | 0,29 |
| | Dioxyde de titane enrobé de stéaroyl glutamate d'aluminium | 10,50 |
| **A5** | Huile de Macadamia | 4,00 |
| | Mélange de tocophérols naturels avec de l'huile de soja (50/50) | 0,50 |
| **A6** | Undécane+Tridécane vendu sous la référence CETIOL UT par la société COGNIS | 13,00 |
| **A7** | Carbonate de calcium synthétique vendu sous la référence SOCAL 90 A par la société SOLVAY | 4,00 |
| **B** | Eau déminéralisée | 32,80 |
| | Glycérol | 5,00 |
| | Chlorure de sodium | 0,60 |
| | Sorbitol en solution aqueuse à 70 % vendu sous la référence NEOSORB 70/70 B par la société ROQUETTE | 2,00 |
| | Alcool benzylique | 1,00 |
| **C** | Silice vendue sous la référence SHERON P1500 par la société CCIC | 4,00 |
| **D** | Ethanol | 5,00 |
| | TOTAL | 100 % |

### Mode opératoire

La composition est préparée comme indiqué à l'Exemple 5.

### Exemple 7 :

### Fond de teint-émulsion E/H

| Composés/références commerciales | | % massique |
|---|---|---|
| **A1** | Di capryl ether vendu sous la référence CETIOL OE par la Société COGNIS | 9,90 |
| **A2** | Hectorite modifiée vendue sous la référence BENTONE 38 VCG par la société ELEMENTIS | 1,00 |
| **A3** | PEG-30 Dipolyhydroxy stearate vendu sous la référence ARLACEL P 135 par la Société UNIQEMA | 5,00 |
| **A4** | Di capryl ether vendu sous la référence CETIOL OE par la Société COGNIS | 5,00 |
| | Oxyde de fer j aune enrobé de stéaroyl glutamate d'aluminium | 2,16 |
| | Oxyde de fer rouge enrobé de stéaroyl glutamate d'aluminium | 0,59 |
| | Oxyde de fer noir enrobé de stéaroyl glutamate d'aluminium | 0,25 |
| | Dioxyde de titane enrobé de stéaroyl glutamate d'aluminium | 9,00 |
| **A5** | Huile de Jojoba | 4,00 |
| | Mélange de tocophérols naturels avec de l'huile de soja (50/50) | 0,50 |
| **A6** | Undécane+Tridécane vendu sous la référence CETIOL UT par la société COGNIS | 8,00 |
| **A7** | Carbonate de calcium synthétique vendu sous la référence SOCAL 90 A par la société SOLVAY | 3,00 |
| **B** | Eau déminéralisée | 34,00 |
| | Glycérol | 5,00 |
| | Chlorure de sodium | 0,60 |
| | Sorbitol en solution aqueuse à 70 % vendu sous la référence NEOSORB 70/70 B par la société ROQUETTE | 2,00 |
| | Alcool benzylique | 1,00 |
| **C** | Silice vendue sous la référence SHERON P1500 par la société CCIC | 4,00 |
| **D** | Ethanol | 5,00 |
| | TOTAL | 100 % |

### Mode opératoire

La composition est préparée comme indiqué à l'Exemple 5.

## Revendications

1. Composition cosmétique sous forme d'une émulsion comprenant au moins une phase aqueuse et au moins une phase grasse, ladite phase grasse contenant au moins une huile végétale, et ladite composition comprenant au moins un solvant volatil de type alcane(s) linéaire(s) volatil(s) en C₉ à C₁₅ et au moins un carbonate de calcium.

2. Composition selon la revendication précédente, dans laquelle ledit solvant volatil de type alcane(s) linéaire(s) volatil(s) comprend au moins un alcane linéaire volatil comprenant de 10 à 15 atomes de carbone, et plus particulièrement de 11 à 13 atomes de carbone.

3. Composition selon l'une quelconque des revendications précédentes, dans laquelle ledit solvant volatil de type alcane(s) linéaire(s) volatil(s) est d'origine végétale.

4. Composition selon l'une quelconque des revendications précédentes, dans laquelle ledit solvant volatil de type alcane(s) linéaire(s) volatil(s) comprend au moins un alcane linéaire volatil choisi parmi le n-nonane, le n-undécane, le n-dodécane, le n-tridécane, et leurs mélanges.

5. Composition selon l'une quelconque des revendications précédentes, comprenant de 0,5 % à 90 % en poids, en particulier de 1 % à 50 % en poids, et plus particulièrement de 5 % à 40 % en poids de solvant volatil de type alcane(s) linéaire(s) volatil(s) par rapport au poids total de la composition.

6. Composition selon l'une quelconque des revendications précédentes, comprenant des craies, des calcaires, des marbres et leurs mélanges à titre de source en carbonate de calcium.

7. Composition selon l'une quelconque des revendications précédentes, dans laquelle le carbonate de calcium est présent sous forme de particules présentant une taille moyenne variant de 0,1 à 20 µm, en particulier de 0,5 à 10 µm, et plus particulièrement de 1 à 5 µm.

8. Composition selon l'une quelconque des revendications précédentes, comprenant de 0,1 % à 20 % en poids, en particulier de 0,5 à 15 % en poids, et plus particulièrement de 1 % à 10 % en poids de carbonate de calcium par rapport au poids total de la composition.

9. Composition selon l'une quelconque des revendications précédentes, dans laquelle ladite huile végétale est choisie parmi l'huile d'aloès, l'huile d'amande douce, l'huile d'amande de pêche, l'huile d'arachide, l'huile d'argan, l'huile d'avocat, l'huile de bancoulier, l'huile de baobab, l'huile de bourrache, l'huile de brocoli, l'huile de calendula, l'huile de caméline, l'huile de carotte, l'huile de carthame, l'huile de chanvre, l'huile de colza, l'huile de coton, l'huile de coprah, l'huile de graine de courge, l'huile de germe de blé, l'huile de jojoba, l'huile de karité, l'huile de luzerne, l'huile de lys, l'huile de macadamia, l'huile de maïs, l'huile de meadowfoam, l'huile de millepertuis, l'huile de millet, l'huile de monoï, l'huile de noisette, l'huile de noyaux d'abricot, l'huile de noix, l'huile d'olive, l'huile d'onagre, l'huile d'orge, l'huile de palme, l'huile de passiflore, l'huile de pavot, l'huile de pépins de cassis, l'huile de pépins de kiwi, l'huile de pépins de raisin, l'huile de pistache, l'huile de potiron, l'huile de potimarron, l'huile de quinoa, l'huile de rosier muscat, l'huile de sésame, l'huile de seigle, l'huile de soja, l'huile de tournesol, l'huile de ricin, et l'huile de watermelon, et leurs mélanges.

10. Composition selon l'une quelconque des revendications précédentes, comprenant de 0,5 % à 80 % en poids, en particulier de 1 % à 50 % en poids, et plus particulièrement de 2 % à 25 % en poids d'huile végétale par rapport au poids total de la composition.

11. Composition selon l'une quelconque des revendications précédentes, comprenant au moins un agent tensioactif à raison de 0,3 à 20 % en poids d'agents tensioactifs, en particulier de 0,5 % à 15 %, et plus particulièrement de 1 % à 10 % en poids d'agents tensioactifs par rapport au poids total de la composition.

12. Composition selon l'une quelconque des revendications précédentes, comprenant en outre au moins une matière colorante.

13. Composition selon l'une quelconque des revendications précédentes, ladite composition étant un fond de teint.

14. Utilisation d'au moins un solvant volatil de type alcane(s) linéaire(s) volatil(s) en association avec au moins un carbonate de calcium dans une émulsion comprenant au moins une huile végétale, pour conférer une stabilité améliorée à ladite émulsion.

15. Procédé cosmétique de maquillage et/ou de soin de matières kératiniques, comprenant au moins l'application sur lesdites matières d'au moins une couche d'une composition telle que définie selon l'une quelconque des revendications 1 à 13.
